# FASCICULE DE BREVET EUROPEEN

(11) **EP 0 652 964 B1**
(45) Date de publication et mention de la délivrance du brevet: **28.12.2005**
(21) Numéro de dépôt: 93917834.9
(22) Date de dépôt: 28.07.1993
(51) Int. Cl.: C12N 15/81, C12N 15/62, C12N 15/14, C12N 15/54, C12N 1/21, C12N 15/12

(54) **PROMOTEUR DU GENE DE LA TRANSALDOLASE DE K. LACTIS ET SON UTILISATION**
PROMOTOR DES TRANSALDOLASE-GENES VON K.LACTIS UND DESSEN VERWENDUNG
K. LACTIS TRANSALDOLASE GENE PROMOTER AND USE THEREOF

(30) Priorité: 30.07.1992 FR 9209432
(43) Date de publication de la demande: 17.05.1995
(73) Titulaire: Aventis Pharma S.A., 92160 Antony (FR)
(72) Inventeur: BOLOTIN, Monique Résidence de Courcelles, F-91190 Gif-sur-Yvette (FR); MENART, Sandrine, F-91940 Les Ulis (FR)
(74) Mandataire: Le Pennec, Magali
(86) Numéro de dépôt international: PCT/FR1993/000771
(87) Numéro de publication internationale: WO 1994/003618

(56) Documents cités:
- EP-A- 0 361 991
- JOURNAL OF BASIC MICROBIOLOGY vol. 28, no. 4, 1988, BERLIN, DE pages 211 - 220 CHEN, X.J. ET AL. 'A gene-cloning system for Kluyveromyces lactis and isolation of a chromosomal gene required for killer toxin production' cité dans la demande
- Banque de données EMBL, fiche KLTRANAL Numéro d'accès Z17317 (version 1); 22 Octobre 1992; JACOBY, J. et al.: ' Transaldolase mutants give evidence for a significant contribution of the pentose phosphate pathway to carbohydrate metabolism in the yeast Kluyveromyces lactis'

## Description

La présente invention concerne le domaine de la biologie moléculaire. Plus particulièrement, elle concerne une nouvelle séquence d'ADN présentant une activité de promoteur transcriptionnel, des vecteurs d'expression contenant cette séquence, et son utilisation pour la production de protéines recombinantes, et par exemple de protéines hétérologues. L'invention concerne aussi les cellules recombinées contenant cette séquence d'ADN.

Les progrès accomplis dans le domaine de la biologie moléculaire ont permis de modifier des microorganismes pour leur faire produire des protéines hétérologues. En particulier, de nombreuses études génétiques ont porté sur la bactérie *E. coli.* Toutefois, l'application industrielle de ces nouveaux modes de production est encore limitée, en particulier par les problèmes d'efficacité d'expression des gènes dans ces microorganismes recombinés. Aussi, dans le but d'augmenter les performances de ces systèmes de production, des recherches ont été effectuées afin d'isoler des promoteurs forts, permettant d'obtenir des niveaux élevés d'expression de protéines hétérologues. Chez *E.coli,* on peut citer en particulier les promoteurs des opérons tryptophane et lactose.

Plus récemment, chez la levure *S.cerevisiae,* des études ont porté sur des promoteurs dérivés de gènes impliqués dans la glycolyse. On peut citer notamment les travaux sur le promoteur du gène de la 3-phosphoglycérate kinase PGK (Dobson et al., Nucleic Acid Res. 10, 1982, 2625; Hitzeman et al., Nucleic Acid Research 1982, 7791), sur celui du gène de la glyceraldéhyde-3-phosphate deshydrogénase GAPDH (Holland et al., J.Biol.Chem. 254, 1979, 9839; Musti et al., Gene 25, 1983, 133), sur celui du gène de l'alcool deshydrogénase 1 ADH1 (Bennentzen et al., J.Biol.Chem. 257, 1982, 3018; Denis et al., J.Biol.Chem. 25, 1983, 1165), sur celui du gène de l'enolase 1 ENO1 (Uemura et al., Gene 45, 1986, 65), sur celui du gène GAL1/GAL10 (Johnston et Davis, Mol. Cell. Biol. 4 (1984) 1440) ou sur celui du gène CYC1 (Guarente et Ptashne, PNAS 78 (1981) 2199).

Récemment, des outils génétiques ont été développés afin de se servir de la levure *Kluyveromyces* comme cellule hôte pour la production de protéines recombinantes. La mise en évidence d'un plasmide de type 2-micron originaire de *K. drosophilarum* (plasmide pKD1 - EP 241 435) a permis d'établir un système hôte/vecteur très efficace pour la production de protéines recombinantes (EP 361 991). Cependant, les promoteurs utilisés dans ce système n'ont pas été optimisés jusqu'à présent. En particulier, il s'agit essentiellement de promoteurs hétérologues, c'est-à-dire provenant d'autres microorganismes, tel que notamment *S.cerevisiae.* Cette situation peut engendrer différents inconvénients, et notamment limiter l'activité du promoteur à cause de l'absence de certains éléments de la machinerie transcriptionnelle (par exemple de trans-activateurs), présenter une certaine toxicité pour la cellule hôte due à une absence de régulation, ou affecter la stabilité du vecteur.

Dans ces conditions, le manque de promoteurs homologues forts chez *Kluyveromyces* constitue un facteur limitant dans l'exploitation industrielle de ce système d'expression.

La Demanderesse a maintenant identifié, cloné et séquencé une région du génome de *Kluyveromyces lactis* présentant une activité de promoteur transcriptionnel (SEQ ID n° 1). Plus précisément, cette région correspond au promoteur d'un gène codant pour une transaldolase de *K. lactis* (désigné gène K1TAL1). Cette région, ou des dérivés ou fragments de celle-ci, peut être utilisée de manière très performante pour la production de protéines recombinantes chez les levures du genre *Kluyveromyces.* Il est entendu que cette séquence peut également être utilisée dans d'autres organismes hôtes.

Par ailleurs, un avantage de la région promotrice obtenue réside dans l'absence de répression par le glucose, permettant une utilisation dans des milieux de culture classiques et industriels.

Un objet de la présente invention réside donc dans une séquence d'ADN comprenant tout ou partie de la séquence SEQ ID n° 1 ou de son brin complémentaire, ou d'un dérivé de celles-ci, et possédant une activité de promoteur transcriptionnel.

Au sens de la présente invention, on entend par dérivé, toute séquence obtenue à partir de la séquence SEQ ID n° 1 par modification(s) de nature génétique et/ou chimique, conservant une activité de promoteur. Par modification de nature génétique et/ou chimique, on entend toute mutation, délétion, substitution, addition, et/ou modification d'un ou plusieurs nucléotides. De telles modifications peuvent être effectuées dans différents buts, et notamment celui de préparer des promoteurs portables, ou celui de préparer des promoteurs adaptés à l'expression dans un type particulier de vecteur ou d'hôte, celui de réduire la taille, d'augmenter l'activité de promoteur de la transciption, de générer des promoteurs inductibles, d'améliorer le niveau de régulation, ou encore de changer la nature de la régulation. De telles modifications peuvent être effectuées par exemple par mutagénèse *in vitro,* par introduction d'éléments additionnels de contrôle ou de séquences synthétiques, ou par des délétions ou des substitutions des éléments originels de contrôle.

Lorsqu'un dérivé tel que défini ci-dessus est réalisé, son activité de promoteur transcriptionnel peut être mise en évidence de plusieurs façons, et en particulier en plaçant sous le contrôle de la séquence étudiée, un gène reporteur dont l'expression est détectable. Toute autre technique connue de l'homme de l'art peut bien évidemment être utilisée à cet effet.

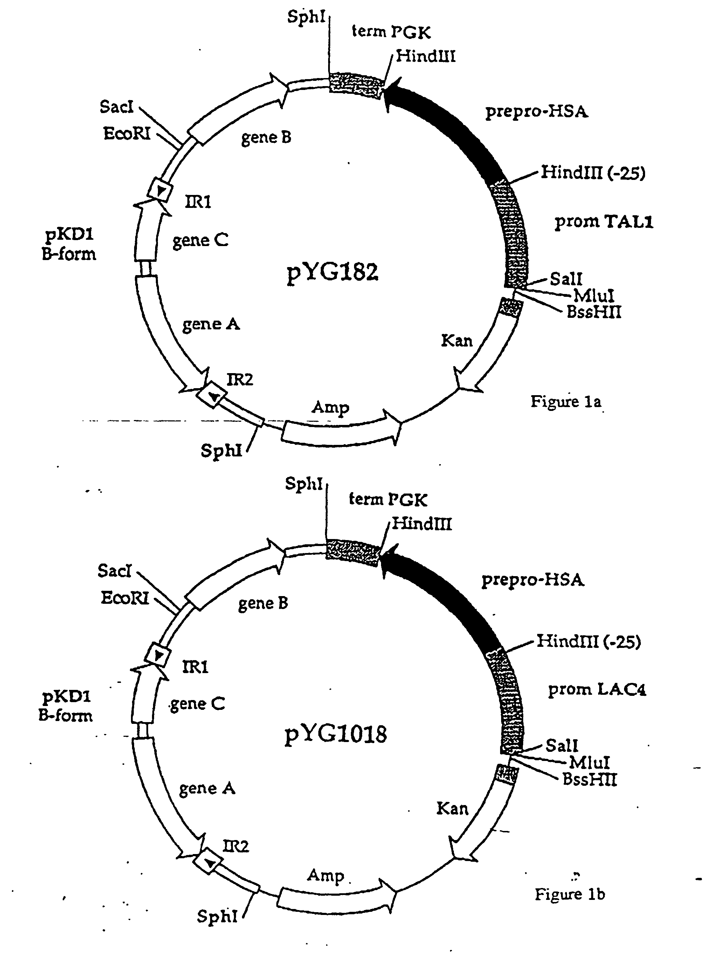

La séquence SEQ ID n° 1a été obtenue à partir d'une banque de fusion entre des fragments du génome de *K. lactis* 2359/152 et le gène lacZ de *E.coli* selon le protocole décrit dans les exemples. Il est entendu que l'homme du métier peut isoler cette région par hybridation au moyen d'une sonde comprenant tout ou partie de la séquence donnée sur la figure 1 ou de son brin complémentaire. Les dérivés selon l'invention peuvent ensuite être préparés à partir de cette séquence, comme indiqué dans les exemples.

Un autre objet de l'invention concerne un ADN recombinant comprenant une séquence d'ADN telle que définie ci-dessus.

Cet ADN recombinant peut contenir par exemple la séquence promotrice SEQ ID n° 1 ou un dérivé de celle-ci, dans laquelle est inséré un site de restriction, facilitant l'utilisation de cette séquence comme promoteur "portable" (Cf SEQ ID n° 3).

Préférentiellement, cet ADN recombinant contient en outre un ou plusieurs gènes de structure. En particulier, il peut s'agir de gènes codant pour des protéines d'intérêt pharmaceutique ou agroalimentaire. A titre d'exemple, on peut citer les enzymes (tels que notamment la superoxide dismutase, la catalase, les amylases, les lipases, les amidases, la chymosine, etc), les dérivés sanguins (tels que la sérum-albumine, l'alpha- ou la béta-globine, le facteur VIII, le facteur IX, le facteur de von Willebrand, la fibronectine, l'alpha-1 antitrypsine, etc), l'insuline et ses variants, les lymphokines (telles que les interleukines, les interférons, les facteurs de stimulation des colonies [G-CSF, GM-CSF, M-CSF...], le TNF, le TRF, etc), les facteurs de croissance (tels que l'hormone de croissance, l'érythroprotéine, le FGF, l'EGF, le PDGF, le TGF, etc), les apolipoprotéines, des polypeptides antigéniques pour la réalisation de vaccins (hépatite, cytomégalovirus, Eppstein-Barr, herpes, etc), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus [CD4, etc.D.

Encore plus préférentiellement, l'ADN recombinant contient également des signaux permettant la sécrétion du produit d'expression du ou desdits gènes de structure. Ces signaux peuvent correspondre aux signaux naturels de sécrétion de la protéine considérée, mais ils peuvent être d'une origine différente. En particulier des signaux de sécrétion dérivés de gènes de levure peuvent être utilisés, tels que ceux des gènes de la toxine killer (Stark et Boyd, EMBO J. 5 (1986) 1995) ou de la phéromone alpha (Kurjan et Herskowitz, Cell 30 (1982) 933 ; Brake et al., Yeast 4 (1988) S436).

Dans un mode de réalisation particulier de l'invention, l'ADN recombinant fait partie d'un plasmide d'expression, qui peut être à réplication autonome ou intégratif.

En particulier, des vecteurs à réplication autonome peuvent être obtenus en utilisant des séquences à réplication autonomes chez l'hôte choisi. Notamment, chez la levure, il peut s'agir d'origines de réplication dérivées de plasmides (pKD1, 2µ, etc), ou bien de séquences chromosomiques (ARS).

Les vecteurs intégratifs peuvent être obtenus notamment en utilisant des séquences homologues à certaines régions du génome de l'hôte, permettant, par recombinaison homologue, l'intégration du vecteur.

Un autre objet de l'invention concerne les cellules recombinées contenant une séquence d'ADN telle que définie ci-avant.

Avantageusement, les cellules sont choisies parmi les levures, et encore plus préférentiellement, parmi les levures du genre *Kluyveromyces.* Il est entendu cependant que l'invention couvre toutes les cellules recombinées dans lesquelles les régions promotrices de l'invention sont actives, qu'il s'agisse de cellules eucaryotes ou procaryotes.

Ainsi, parmi les cellules eucaryotes, on peut citer les cellules végétales, animales, les levures, ou les champignons. En particulier, s'agissant de levures, on peut citer les levures du genre *Saccharomyces, Pichia, Schwanniomyces,* ou *Hansenula.* S'agissant de cellules animales, on peut citer les cellules COS, CHO, C127, etc. Parmi les champignons susceptibles d'être utilisés dans la présente invention, on peut citer plus particulièrement *Aspergillus* ssp. ou *Trichoderma* ssp. Comme hôtes procaryotes, on peut utiliser les bactéries telles que *Escherichia coli,* ou celles appartenant aux genres *Corynebacterium, Bacillus,* ou *Streptomyces.*

L'activité de promoteur de la transcription des séquences de l'invention dans ces différents hôtes peut être vérifiée par exemple en introduisant dans la cellule hôte considérée un ADN recombinant comprenant, sous le controle de la séquence promotrice étudiée, un gène reporteur dont l'expression peut être mise en évidence dans l'hote considéré.

Les cellules recombinées de l'invention peuvent être obtenues par toute méthode permettant d'introduire un ADN étranger dans une cellule. Il peut s'agir notamment de transformation, électroporation, conjugaison, fusion de protoplastes, ou toute autre technique connue de l'homme de l'art. S'agissant de la transformation, différents protocoles ont été décrits dans l'art antérieur. En particulier, elle peut être réalisée en traitant les cellules entières en présence d'acétate de lithium et de polyéthylène glycol selon la technique décrite par Ito et al. (J. Bacteriol. 153 (1983) 163-168), ou en présence d'éthylène glycol et de diméthylsulfoxyde selon la technique de Durrens et al. (Curr. Genet. 18 (1990) 7). Un protocole alternatif a également été décrit dans la demande de brevet EP 361 991. S'agissant d'électroporation, elle peut être réalisée selon Becker et Guarentte (in : Methods in Enzymology Vol194 (1991) 182).

Un autre objet de l'invention concerne l'utilisation d'une séquence telle que précédemment définie pour l'expression de gènes recombinés. Les séquences d'ADN selon l'invention peuvent en effet permettre une production à des niveaux élevés de protéines recombinantes.

Avantageusement, les séquences de l'invention peuvent être utilisées pour l'expression de gènes codant pour des protéines d'intérêt pharmaceutique ou agroalimentaire. A titre d'exemple, on peut citer les protéines énumérées précédemment.

La présente invention permet également la réalisation d'un procédé de production de protéines recombinantes, selon lequel on cultive une cellule recombinée telle que définie ci-avant et on récupère la protéine produite. A titre d'exemple de protéine, on peut citer les protéines énumérées précédemment.

Préférentiellement, le procédé de l'invention est applicable à la production de sérum-albumine humaine, ou un de ses variants moléculaires. On entend par variant moléculaire de l'albumine, les variants naturels résultant du polymorphisme de l'albumine, les formes tronquées, ou toute protéine hybride à base d'albumine.

D'autres avantages de la présente invention apparaitront à la lecture des exemples qui suivent, qui doivent être considérés comme illustratifs et non limitatifs.

### LEGENDE DES FIGURES

Figure 1 : Carte de restriction des plasmides pYG1018 et pYG182.
   SEQ ID n°1 Séquence nucléotidique du fragment de 1,3 kb correspondant au promoteur K1TAL1 de *K*. *lactis.*
Figure 2 : Préparation du transposon Mini Mu MudIIZK1.
Figure 3 : Carte de restriction du transposon Mini Mu MudIIZK1.
Figure 4 : Carte de restriction du clone 12C4.
Figure 5: Carte de restriction du fragment BamHI-HindIII de 2,5 kb portant le promoteur K1TaL1.

### TECHNIQUES GENERALES DE CLONAGE

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans *Escherichia coli* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les enzymes de restriction ont été fournies par New England Biolabs (Biolabs), ou Pharmacia et sont utilisées selon les recommandations des fournisseurs.

Les plasmides de type pBR322 et pUC sont d'origine commerciale (Bethesda Research Laboratories).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Boehringer) selon les recommandations du fournisseur.

Le remplissage des extrémités 5' proéminentes est effectué par le fragment de Klenow de l'ADN Polymérase I d'E. *coli* (Boehringer) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques est effectuée selon la méthode développée par Taylor et al. [Nucleic Acids Res. 13 (1985) 8749-8764].

L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467].

Les transformations de *K. lactis* sont effectuées par toute technique connue de l'homme de l'art, et dont un exemple est donné dans le texte.

Sauf indication contraire, les souches bactériennes utilisées sont *E.coli* DH1 (Hanahan D., J. Mol. Biol. 166 (1983) 557) ou Ecoli JM109::(Mucts) (Daignan-fornier et Bolotin-Fukuhara, Gene 62 (1988) 45).

Les souches de levures utilisées appartiennent aux levures bourgeonnantes et plus particulièrement aux levures du genre *Kluyveromyces.* Les souche *K*. *lactis* 2359/152 et *K. lactis* SD6 ont été particulièrement utilisées.

Les souches de levures transformées par les plasmides sont cultivées en erlenmeyers ou en fermenteurs pilotes de 21 (SETRIC, France) à 28°C en milieu riche (YPD: 1 % extrait de levure, 2 % Bactopeptone, 2% glucose; ou YPL: 1 % extrait de levure, 2 % Bactopeptone, 2 % lactose) sous agitation constante.

### EXEMPLES

### I - Isolement du promoteur KITAL1 de K. lactis.

La séquence SEQ ID n° 1 a été isolée à partir d'une banque de fusion entre des fragments du génome de *K. lactis* 2359/152 et le gène lacZ de *E.coli.* Cet exemple décrit en (A) la préparation de la banque de fusion, et en (B) la sélection et la caractérisation d'un clone de cette banque portant le promoteur du gène de la transaldolase TAL1 de *K. lactis.*

### A/ Préparation de la banque de fusion

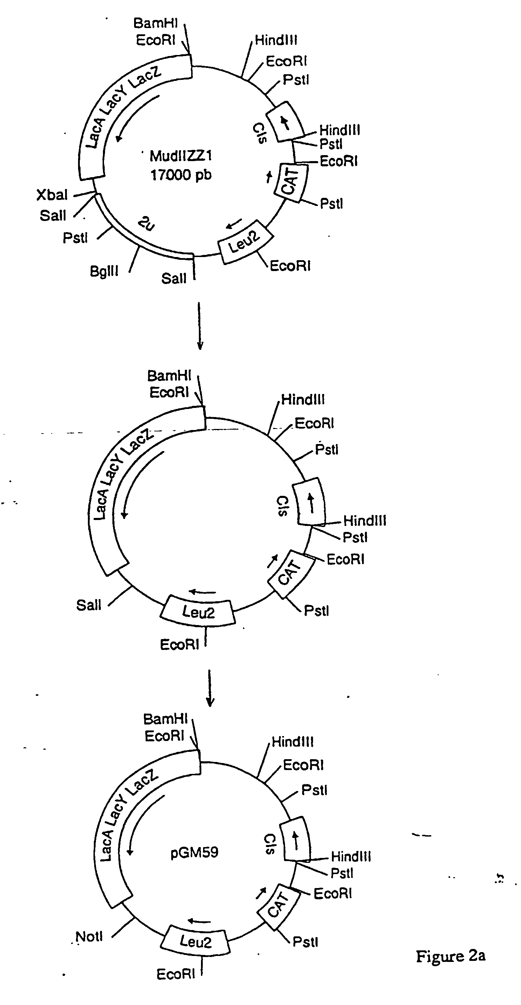

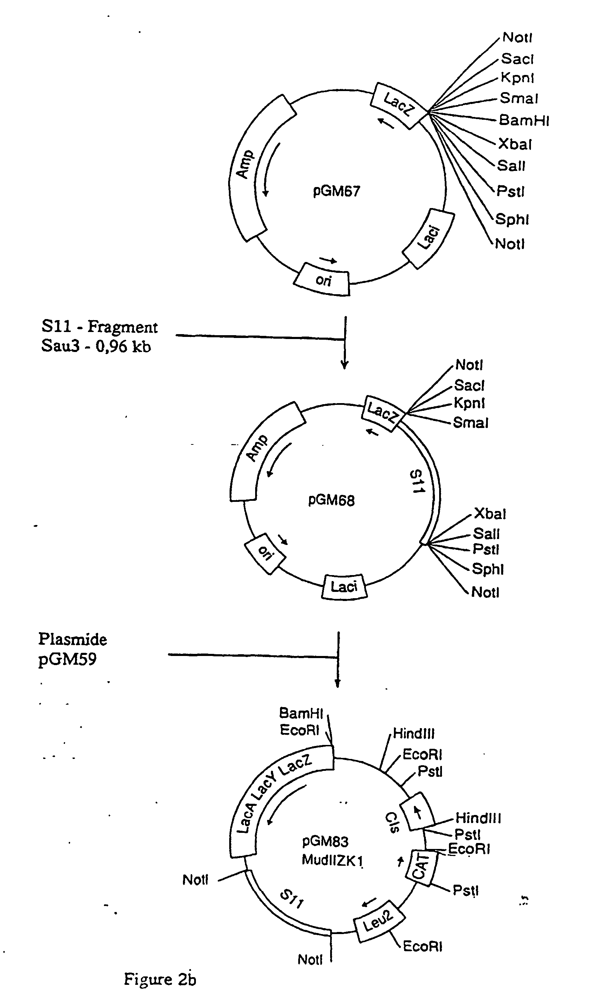

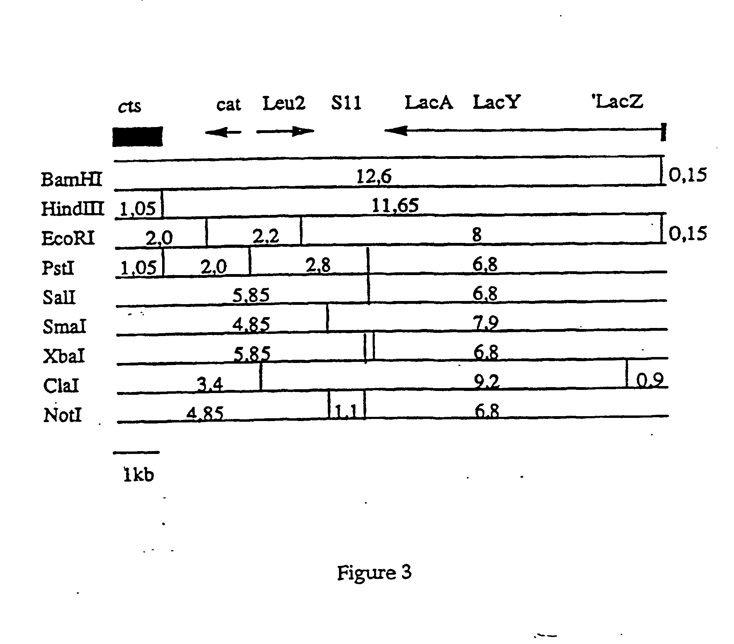

### A.1. Préparation du transposon Mini Mu MudIIZK1 (figures 2 et 3).

Le Mini Mu MudIIZK1 a été construit à partir du Mini Mu MudIIZZ1 décrit par Daignan-Fornier et Bolotin-Fukuhara (Gene 62 (1988) 45). Il a été obtenu en substituant l'origine de réplication du mini transposon MudIIZZ1 par une origine de réplication fonctionnelle dans *Kluyveromyces* : l'origine de réplication du plasmide pKD1 (EP231 435).

### A.1.1. Construction d'une cassette portant l'origine de réplication du plasmide pKD1 (fragment S11).

Afin de faciliter les manipulations ultérieures, le fragment S11 (portant l'origine de réplication du plasmide pKD1) a été mis sous forme d'une cassette NotL Pour cela, un dérivé du plasmide pUC18 a été construit dans lequel les sites externes du multisite de clonage (sites HindIII et EcoRI) ont été changés en sites NotI. Cela a été fait par digestion avec l'enzyme correspondante, action de l'enzyme de Klenow et ligature avec un oligonucléotide synthétique correspondant à un site NotI [oligo d(AGCGGCCGCT); Biolabs]. Le plasmide obtenu est désigné pGM67. Le fragment S11 de 960 pb obtenu par digestion par l'enzyme Sau3A du plasmide KEp6 (Chen et al, Nucl. Acids Res. 114 (1986) 4471) a ensuite été inséré au site compatible BamHI du plasmide pGM67. Le plasmide ainsi obtenu désigné pGM68 contient, sous forme d'une cassette NotI, le fragment S11.

### A.1.2. Suppression de l'origine de réplication 2µ du transposon MudIIZZ1.

Le plasmide pGM15 portant le mini Mu MudIIZZ1 (Daignan-Fornier et Bolotin-Fukuhara précitée) a été délété des régions 2µ par digestion au moyen de l'enzyme SalI. Le site SalI unique ainsi obtenu a ensuite été transformé en site NotI par ligature d'un oligonucléotide synthétique correspondant à un site NotI après action de l'enzyme de Klenow. Le plasmide résultant est appelé pGM59.

### A.1.3. Insertion du fragment S11

La cassette NotI portant l'origine de réplication du plasmide pKD1 (fragment S11), provenant du plasmide pUC18 modifié a ensuite été introduite au site NotI unique du plasmide pGM59.

Le plasmide obtenu, désigné pGM83, porte un mini Mu, appelé MudIIZK1, qui est adapté à la levure *Kluyveromyces lactis,* ainsi qu'une copie fonctionnelle du gène LEU2 de *S.cerevisiae* capable de complémenter une mutation leu2 chez *K.lactis* (Kämper et al, Curr. Genet. 19 (1991) 109). La carte de restriction du mini-mu MudIIZK1 est représentée sur la figure 3.

### A.2. Introduction du Mini Mu MudIIZK1 dans la souche E.coli portant le Mu helper JM109::(Mucts) : Obtention de la souche JM109::(Mucts)::(MudIIZK1).

La souche JM109::(Mucts) a été transformée par le plasmide pGM83 contenant le mini mu MudIIZK1 en présence de chlorure de calcium. Après transformation, la transposition a été induite par choc thermique selon la technique décrite par Castilho et al. (J. Bacteriol 158 (1984) 488). Le lysat phagique obtenu aprés induction est ensuite utilisé pour surinfecter la souche JM109::(Mucts). La souche JM109::(Mucts) étant recA, l'ADN linéaire encapsidé par le phage ne peut se refermer pour donner un plasmide réplicatif. Les intégrants [souche JM109::(Mucts)::(MudIIZK1)] sont donc sélectionnés comme clones chloramphénicol résistants (Cm^{R}). ampicilline sensibles (Amp^{S}).

### A.3. Préparation de la banque génomique de K.lactis dans E.coli DH1

L'ADN de haut poids moléculaire a été préparé à partir de la souche *K.lactis* 2359/152, et digéré partiellement par l'enzyme Sau3A. Les fragments d'une taille de 4 à 8 kb ont été récupérés sur gel d'agarose LMP ("Low Melting Point", SEAXEM) et clonés dans le plasmide pBR322 linéarisé par BamHI et déphosphorylé par action de la phosphatase intestinale de veau (Biolabs). 35 pools de 1000 colonies dans *E*.*coli* DH1 ont ainsi été réalisés. Les 1000 colonies de chaque pool sont ampicilline-résistantes et tétracycline-sensibles, ce qui montre qu'elles ont toutes inséré un fragment d'ADN génomique de *K.lactis* dans pBR322.

### A.4. Préparation de la banque de fusion

### A.4.1. Introduction de la banque génomique de K.lactis dans la souche JM109::(Mucts)::(MudIIZK1).

L'ADN plasmidique de chaque pool réalisé dans DH1 est extrait (Maniatis). Cet ADN est ensuite utilisé pour transformer la souche JM109::(Mucts)::(MudIIZK1) en présence de chlorure de calcium. Pour être représentatif des 1000 colonies contenues dans chaque pool de la banque génomique. plus de 3000 clones par pool ont été récupérés dans la souche JM109::(Mucts)::(MudBZK1) permettant la transduction.

### A.4.2. Transposition du Mini Mu MudIIZK1

La banque de fusion est réalisée par transposition extensive du Mini Mu MudIIZK1 sur les plasmides formant la banque d'ADN génomique de *K.lactis.* Les mini-muductions ont été faites selon le protocole décrit par Castilho et al. (J. Bacteriol. 158 (1984) 488) et les transductants ont été sélectionnés sur milieu sélectif LBAC (milieu LB (Gibco BRL) supplémenté avec 50 mg/l d'ampicilline et 30 mg/l de chloramphénicol), le marqueur Amp^{R} étant apporté par le plasmide, et le marqueur Cm^{R} par le mini-mu. Pour chaque pool, des transpositions sont faites en série, et entre 10 000 et 20 000 transductants sont récupérés par pool. L'ADN des transductants est ensuite extrait d'une préparation de 100 ml, purifié par précipitation au polyéthylène glycol (Maniatis et al, 1989) et resuspendu dans 100µl d'eau. Cet ADN a ensuite été utilisé pour transformer *K.lactis* et sélectionner des clones portant des promoteurs.

### B/ Isolement du promoteur KlTAL1 de K.lactis

L'ADN de fusion préparé ci-dessus a été utilisé pour transformer, par électroporation, une souche réceptrice de *K.lactis.* Cette souche réceptrice, désignée SD6, porte les mutations leu2 (correspondant au marqueur de sélection du mini-mu MudIIZK1) et lac4-8. Cette dernière mutation empêche la souche de pousser sur un milieu contenant du lactose comme seule source de carbone, mais elle peut être complémentée par la surexpression du gène lacZ *d'E.coli* codant pour la β-galactosidase (Chen et al., J. Basic Microbiol. 28 (1988) 211). De ce fait, l'expression d'une protéine fusionnée à la ß-galactosidase doit permettre la croissance de la souche SD6 sur lactose après transformation. Ce crible positif a été utilisé pour sélectionner rapidement des clones portant des promoteurs forts.

### B.1. Construction de la souche réceptrice K. lactis SD6.

La souche SD6 (Chen et al., Mol. Gen. Genet. 233 (1992) 97) a été obtenue par croisement de la souche *K.lactis* CXJ1-7A (a, lac4-8, ura3A, adel-1, K1, K2, pKD1) (Chen et Fukuhara, Gene 69 (1988) 181) avec la souche AWJ-137 (leu2, trp1, homothallique) (Kämper et al, Curr. Genet. 19 (1991) 109), et sélection des spores ayant le génotype ADE⁺, uraA, leu2, lac4-8. Comme les spores obtenues n'étaient pas capables de régénérer après tranformation par protoplastes, un croisement retour a été fait avec la souche CXJ1-7A. Après sporulation en masse, les spores du génotype choisi ont été testées par transformation au chlorure de lithium avec le plasmide KEp6 selon une technique dérivée de celle décrite par Ito et al. (J. Bacteriol. 153 (1983) 163) (la concentration en LiCl est de 20 mM, soit 10 fois moins que celle utilisée par Ito pour *S.cerevisiae).* La souche CXJ1-7A a servi de témoin de transformation.

La souche SD6, sélectionnée sur ces critères, se transforme correctement : 1 à 3. 10⁴ transformants par µg d'ADN; et les transformants ont une stabilité satisfaisante : 30 à 40% des colonies gardent le phénotype [Ura⁺] après 6 générations en milieu non sélectif.

### B.2. Isolement du promoteur K1TAL1.

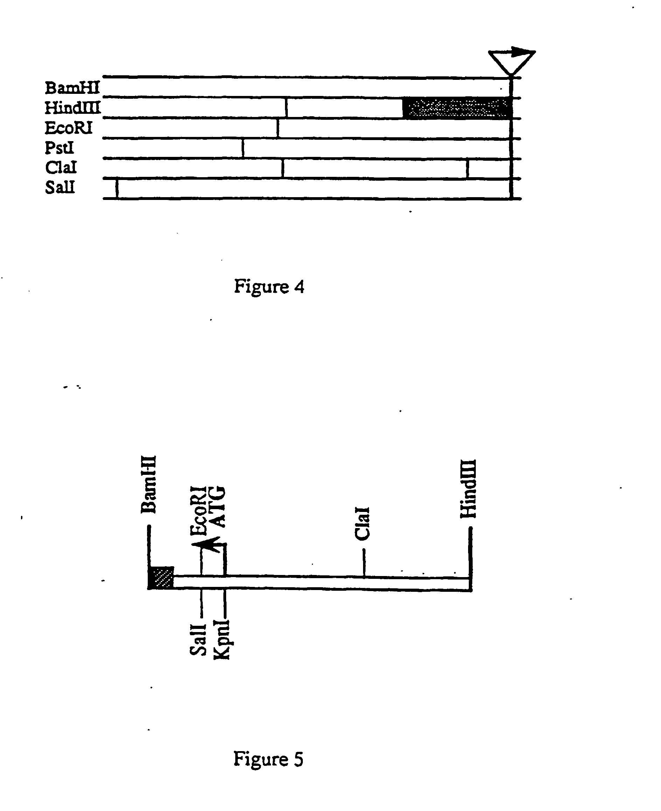

La souche SD6 a été transformée par électroporation selon Becker et Guarante (in Methods in Enzymology vol194 (1991) 182) (appareil Jouan; 2500 V/cm; 80-100 ng d'ADN/transformation) avec l'ADN de 11 pools de transductants obtenus en A.4.2. (correspondant à une banque de 11000 clones dans *E.coli*). Après 5 heures de régénération en milieu YPD (extrait de levure 10 g/l; peptone 10 g/l; glucose 20 g/l), les cellules ont été étalées sur milieu minimum lactose. Les transformants capables de pousser sur lactose ont été restriés et, pour chaque clone, le plasmide a été extrait, amplifié dans *E.coli,* et, après vérification rapide de la carte de restriction du vecteur et du mini-mu, utilisé pour retransformer la levure SD6. Parmi les clones de *K.lactis* obtenus après retransformation, l'un d'entre-eux, le clone 12C4, a été étudié par restriction (voir figure 4) et par analyse de la séquence de la jonction entre la protéine de *K.lactis* et la β-galactosidase. Pour cela, la séquence de la jonction, à partir de l'extrémité lacZ du mini-mu (séquence double brin) a été déterminée par séquençage, au moyen de l'oligonucléotide suivant situé à -59 nucléotides de la jonction :

L'analyse de la séquence protéique déduite de la séquence nucléotidique ainsi obtenue par comparaison avec les séquences de banques de protéines d'autres levures ou eucaryotes (Genbank, MIPS, EMBL, etc), montre que la séquence portée par le clone 12C4 correspond au promoteur du gène de la transaldolase TAL1 de *K. lactis.* Le fragment BamHI-HindIII de 2,5 kb contenant la région en amont de la fusion a ensuite été sous-cloné dans le vecteur Bluescript KS+ (Stratagène), une carte de restriction a été faite (figure 5), et la séquence a été déterminée par délétions séquentielles sur 1,3 kb environ (SEQ ID n° 1). L'obtention d'éléments de séquence permet également à l'homme du métier de préparer des sondes spécifiques et de recloner la région promotrice de l'invention par hybridation selon les techniques classiques de biologie moléculaire.

### II Transformation de Kluyveromyces.

Différentes techniques permettant l'introduction d'ADN dans la levure peuvent être utilisées.

Avantageusement, les différentes souches de *Kluyveromyces* utilisées ont été transformées en traitant les cellules entières en présence d'acétate de lithium et de polyéthylène glycol, selon la technique décrite par Ito et al. (J. Bacteriol. 153 (1983) 163-168). La technique de transformation décrite par Durrens et al. (Curr.Genet. 18 (1990) -7) utilisant l'éthylène glycol et le diméthylsulfoxyde a également été utilisée. Il est aussi possible de transformer les levures par électroporation, par exemple selon la méthode décrite par Karube et al. (FEBS Letters 182 (1985) 90).

Un protocole alternatif a encore été décrit en détail dans la demande EP 361 991.

### III - Utilisation du promoteur de la figure 1 pour l'expression de gènes hétérologues.

L'activité de promoteur transcriptionnel de la région de *K. lactis* décrite sur la SEQ ID n° 1 a été mise en évidence lors même de son isolement, par sa capaciter à induire la complémentation de la mutation lac4-8 de la souche SD6. Cette capacité résulte en effet de l'expression du gène lacz de *E.coli,* et démontre par là même la capacité d'expression de gènes hétérologues.

### IV - Construction d'un promoteur KITAL1 portable

Un promoteur portable a été préparé par PCR en insérant, sur le fragment BamHI-HindIII de 2,5 kb, un site de restriction HindIII en position +1 par rapport au codon,ATG du gène *KITAL1* et des sites de restriction MluI et SalI à 1197 pb en amont (SEQ ID n° 3). Le produit de PCR est cloné dans le vecteur pCRII (Kit TA Cloning™ System, Invitrogen). obtenant ainsi le vecteur pYG176. Cette construction permet de ressortir le promoteur *KITAL1* par digestion MluI-HindIII et facilite le clonage dans les sites correspondants du vecteur d'expression pYG1018 qui contient le gène prepro-albumine humaine sous contrôle du promoteur *LAC4.* Le plasmide pYG1018 est identique au vecteur pYG1023 décrit dans le brevet EPA 402 212,sauf qu'il ne contient pas le fragment BssHII-MluI portant le gène *KlPGK.*

5 µg des vecteurs pYG176 et pYG1018 sont digérés par 60 unités de HindIII et de MluI. Après migration sur gel d'agarose à 0,8 %, la bande d'environ 1,2 kb pour pGY176 (correspondant au promoteur *KlTAL1*) et les bandes d'environ 9 kb (correspondant à la partie vecteur) et de 2 kb (correspondant au fragment albumine) pour pYG1018 sont électroéluées. Une ligation à trois partenaires (suivant les recommandations de tampon et de température définies par le fournisseur, New England Biolabs) est ensuite effectuée. La préparation de plasmides des transformants obtenus après transformation de *E*. *coli* suivant la technique décrite par Chung et Miller [Nucleic Acids Res., 16 (1988) 3580], suit la méthode de lyse alcaline au SDS de Bimboim et Doly [Nucleic Acids Res., 6 (1979) 1513] modifiée par Ish-Horowicz et Burke (Nucleic Acids Res., 9 (1981) 2989]. Après digestion enzymatique, le plasmide possédant le bon profil de restriction est désigné pYG182 (figure 1).

Une transformation de la souche CBS293.91 de *K*. *lactis* selon le protocole décrit par Durrens et al. [Curr. Genetics, 18 (1990) 7] par 10 µg de pYG182 est effectuée. Les transformants sont sélectionnés sur milieu YPD (2 % de glucose, 1 % d'extrait de levure, 2 % de bacto-peptone, 1,5 % de bacto-agar) supplémenté avec l'antibiotique généticine (200 µg/ml).

La production d'albumine par plusieurs transformants de la souche contenant pYG182 est testée selon la méthode décrite dans les demandes de brevet EPA 361991 et EPA 521 767. La quantité d'albumine sécrétée par les différents transformants est similaire et on peut l'estimer à environ 50 mg/litre.

## Revendications

1. Séquence d'ADN comprenant tout ou partie de la séquence SEQ ID N° 1, ladite partie possédant une activité de promoteur transcriptionnel.

2. ADN recombinant comprenant une séquence d'ADN selon la revendication 1.

3. ADN recombinant selon la revendication 2 de séquence SEQ ID n° 3.

4. ADN recombinant selon la revendication 2 ou 3 **caractérisé en ce qu'**il contient en outre un ou plusieurs gènes de structure.

5. ADN recombinant selon la revendication 4 **caractérisé en ce qu'**il contient également des signaux permettant la sécretion du produit d'expression du ou desdits gènes de structure.

6. ADN recombinant selon les revendications 3 à 5 **caractérisé en ce que** le ou les gènes de structure codent pour des protéines d'intérêt pharmaceutique ou agroalimentaire.

7. ADN recombinant selon la revendication 6 **caractérisé en ce que** le ou les gènes de structure codent pour des protéines choisies parmi les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.).

8. ADN recombinant selon l'une quelconque des revendications 2 à 7 **caractérisé en ce qu'**il fait partie d'un plasmide d'expression, qui peut être à réplication autonome ou intégratif.

9. Cellule recombinée contenant un ADN recombinant selon la revendication 8.

10. Cellule recombinée selon la revendication 9 **caractérisée en ce qu'**il s'agit d'une levure.

11. Cellule recombinée selon la revendication 10 **caractérisée en ce qu'**il s'agit d'une levure du genre Kluyveromyces.

12. Utilisation d'une séquence d'ADN selon l'une quelconque des revendications 1 à 8 pour l'expression de gènes recombinés.

13. Utilisation selon la revendication 12 pour l'expression de gènes codant pour des protéines d'intérêt pharmaceutique ou agroalimentaire.

14. Procédé de production de protéines recombinantes **caractérisé en ce que** l'on cultive une cellule recombinée selon l'une quelconque des revendications 9 à 11 et on récupère les protéines produites.

15. Procédé selon la revendication 14 pour la production de protéines d'intérêt pharmaceutique ou agroalimentaire.

16. Procédé selon la revendication 15 **caractérisé en ce que** la protéine est préférentiellement la sérum-albumine humaine ou un de ses variants moléculaires.

## Patentansprüche

1. DNA-Sequenz, die Sequenz SEQ ID NO: 1 ganz oder einen Teil davon umfasst, wobei der genannte Teil Transkriptionspromotor-Aktivität besitzt.

2. Rekombinante DNA, die eine DNA gemäß Anspruch 1 umfasst.

3. Rekombinante DNA nach Anspruch 2 mit SEQ ID NO: 3.

4. Rekombinante DNA nach Anspruch 2 oder 3, **dadurch gekennzeichnet, dass** sie außerdem ein Strukturgen oder mehrere Strukturgene enthält.

5. Rekombinante DNA nach Anspruch 4, **dadurch gekennzeichnet, dass** sie auch Signale enthält, die die Sekretion des Expressionsproduktes des Strukturgens oder der Strukturgene ermöglichen.

6. Rekombinante DNA nach den Ansprüchen 3 bis 5, **dadurch gekennzeichnet, dass** das Strukturgen oder die Strukturgene für Proteine von pharmazeutischem oder agroalimentärem Interesse codiert/codieren.

7. Rekombinante DNA nach Anspruch 6, **dadurch gekennzeichnet, dass** das Strukturgen oder die Strukturgene für Proteine, ausgewählt unter Enzymen (wie insbesondere Superoxiddismutase, Katalase, Amylasen, Lipasen, Amidasen, Chymosin usw.), Blutderivaten (wie z.B. Serumalbumin, alpha-oder beta-Globin, Faktor VIII, Faktor IX, von Willebrand-Faktor, Fibronectin, alpha-1-Antitrypsin usw.), Insulin und seinen Varianten, Lymphokinen (wie z.B. Interleukine, Interferone, Kolonien stimulierende Faktoren (G-CSF, GM-CSF, M-CSF ...), TNF, TRF usw.), Wachstumsfaktoren (wie z.B. Wachstumshormon, Erythropoietin, FGF, EGF, PDGF, TGF usw.), Apolipoproteinen, antigenen Polypeptiden zur Herstellung von Vakzinen (Hepatitis, Cytomegalovirus, Eppstein-Barr, Herpes usw.) oder auch von Fusionen von Polypeptiden, wie insbesondere Fusionen, die einen aktiven Teil fusioniert mit einem stabilisierenden Teil umfassen (z.B. Fusionen zwischen Albumin oder Albuminfragmenten und dem Virusrezeptor oder einem Teil eines Virusrezeptors (CD4 usw.)), codiert/codieren.

8. Rekombinante DNA nach einem der Ansprüche 2 bis 7, **dadurch gekennzeichnet, dass** sie zu einem Expressionsplasmid gehört, das zur autonomen oder integrativen Replikation fähig ist.

9. Rekombinante Zelle, die eine rekombinante DNA nach Anspruch 8 enthält.

10. Rekombinante Zelle nach Anspruch 9, **dadurch gekennzeichnet, dass** es sich um eine Hefe handelt.

11. Rekombinante Zelle nach Anspruch 10, **dadurch gekennzeichnet, dass** es sich um eine Hefe der Gattung Kluyveromyces handelt.

12. Verwendung einer DNA-Sequenz nach einem der Ansprüche 1 bis 8 zur Expression von rekombinierten Genen.

13. Verwendung nach Anspruch 12 zur Expression von Genen, die für Proteine von pharmazeutischem oder agroalimentärem Interesse codieren.

14. Verfahren zur Herstellung von rekombinanten Proteinen, **dadurch gekennzeichnet, dass** man eine rekombinante Zelle nach einem der Ansprüche 9 bis 11 kultiviert und die produzierten Proteine isoliert.

15. Verfahren nach Anspruch 14, zur Herstellung von Proteinen von pharmazeutischem oder agroalimentärem Interesse.

16. Verfahren nach Anspruch 15, **dadurch gekennzeichnet, dass** das Protein vorzugsweise humanes Serumalbumin oder eine seiner molekularen Varianten ist.

## Claims

1. DNA sequence comprising all or part of the sequence SEQ ID No. 1, the said part having a transcriptional promoter activity.

2. Recombinant DNA comprising a DNA sequence according to Claim 1.

3. Recombinant DNA according to Claim 2 of the sequence SEQ ID No. 3.

4. Recombinant DNA according to Claim 2 or 3, **characterized in that** it also contains one or more structural genes.

5. Recombinant DNA according to Claim 4, **characterized in that** it also contains signals which allow secretion of the expression product of the said structural gene or genes.

6. Recombinant DNA according to Claims 3 to 5, **characterized in that** the structural gene or genes code for proteins of pharmaceutical or agro-nutritional interest.

7. Recombinant DNA according to Claim 6, **characterized in that** the structural gene or genes code for proteins chosen from enzymes (such as, in particular, dismutase superoxide, catalase, amylases, lipases, amidases, chymosin and the like), blood derivatives (such as serum albumin, alpha- or beta-globin, factor VIII, factor IX, von Willebrand factor, fibronectin, 1-alpha-antitrypsin and the like), insulin and its variants, lymphokines (such as interleukins, interferons, colony stimulating factors (G-CSF, GM-CSF, M-CSF...), TNF, TRF and the like), growth factors (such as growth hormone, erythropoietin, FGF, EGF, PDGF, TGF and the like), apolipoproteins, antigenic polypeptides for realization of vaccines (hepatitis, cytomegalovirus, Eppstein-Barr, herpes and the like) or also fusions of polypeptides, such as, in particular, fusions comprising an active part fused to a stabilizing part (for example fusions between albumin or albumin fragments and the virus receptor or a part of a virus receptor (CD4 and the like)).

8. Recombinant DNA according to any one of Claims 2 to 7, **characterized in that** it is part of an expression plasmid, which may be of autonomous or integrated replication.

9. Recombinant cell containing a recombinant DNA according to Claim 8.

10. Recombinant cell according to Claim 9, **characterized in that** it is a yeast.

11. Recombinant cell according to Claim 10, **characterized in that** it is a yeast of the genus Kluyveromyces.

12. Use of a DNA sequence according to any one of Claims 1 to 8 for expression of recombinant genes.

13. Use according to Claim 12 for expression of genes coding for proteins of pharmaceutical or agro-nutritional interest.

14. Process for the production of recombinant proteins, **characterized in that** a recombinant cell according to any one of Claims 9 to 11 is cultured and the proteins produced are recovered.

15. Process according to Claim 14 for the production of proteins of pharmaceutical or agro-nutritional interest.

16. Process according to Claim 15, **characterized in that** the protein is preferably human serum albumin or one of its molecular variants.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Promoteur de levure et son utilisation
   (iii) NOMBRE DE SEQUENCES: 3
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1 :
   (i) CARACTERISTIQUES DE LA SEQUENCE:
      (A) LONGUEUR: 1349 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Kluyveromyces lactis
   (ix) CARACTERISTIQUE ADDITIONELLE:
      (A) NOM/CLE: CDS
      (B) EMPLACEMENT: 1297.. 1349
   (xi) DESCRIPTION DE LA SEQUENCE: SEQ ID NO: 1:
   CCCCTATCAC ATGTAATGTA TTAAAATCTC TAATGTCAAA TTTCCCAGCA TCCAAAGGTT 60
   CTCAAGGTTC AATCCTTCAC TTCTGCTACC TAATCCACAG GTGCCTTTGT ATAACTCCAC 120
   TAATTCGCTA CATTTTTCAT CTCTTTGATT CGCTTTCATT AATAAAATAT TACTATTCGA 180
   GTTGCTATAA CTTTTAAGAT CATATACTTG TGTATATATG TTAGTTTGAT GCTTTCTGCA 240
   TTTTTCTTTA TTTCCCTATA GTTTCTTCTG ATATTCTAAT CTGGCGACAG ATACTGGGAC 300
   AAACACGTAA ATCACACACC ATGTCACGTG GTTAGAGAAC AAAAGTGAAA TATGTGTTGG 360
   TGACTGAATA AAATGATGAA GGGTGTTTTG CTTAAAAACG GTGAATAAGT TACCCGGCTG 420
   ATCTGAATAG AATCGATTTT CTGCTTCTCA GCTTCCTATA ATCAAGAAAA ACGCCATGAC 480
   CGGGTAACAT GAATTTTTGG GTGTCGTTAT ACAGAGCATT GCCATCGACA ATGAACCAGG 540
   ATGCAATCTA AGGTGAATTT GAATAAAATA TAGCTCCATC TACGACCAGG CAAGGATCAC 600
   TGGAAAGTCA TCCCGCATAA ACTACCCGGT TGTCCATCCG ATAAAATGAG TGCCATCTAT 660
   AAATCACTTT CTTTCTTACA AACTAGTCTG TTTTTTCCTC CATTATGTAA CCATTTAAAC 720
   GTTAATTCCC CTAATGCGTC TGAATTACTC TCTGTTCAAA ACAAGAAACG AAAATCCGTA 780
   CTCACAAACC AGCGATTCCC ATACATGCAT ACAATGATTT CATAACAGTT TGACTATTGC 840
   TCAGCAGGCG TGAATCAACA CTTTGGCCCT GTCTAACGTT TACGTATGCT ATGTTCTTCT 900
   CTCTTAGTTG TATTGGGTTT TTCCGTGGCT TTCCTCATTT CCTACGGCCA TTTCTCCCTT 960
   TCCCAGATTT TTTTCTTTCT TTGTTCTTCT TCCTGTTATA TAATCCGGAT TTATCACAGT 1020
   ATACAACTTT TCAAACTTTT GATTTATTCA GAATTGGAAT GATGAAATAA TATATTTGGT 1080
   ATATATATAT AAATCAAAGG AAAAAGGTTA ACATTGAGTT TTACAAAGTG TTTTCTTCTT 1140
   TTGCCAATTC AGCTAAATTC TTAATCGGAT CTAATTTTTG TTTAGTAAGA TTCTTTATTT 1200
   TGAGAAGGTT ATTGTCTATT CTAATCTTGG TTATTTCATT CACCAGGAAA CAGTAATTAC 1260
   ACTCAAGTAA TTTACCTTAT AAATCCTACT ACAAGC ATG TCT GAA CCA AGT GCT 1314
   Met Ser Glu Pro Ser Ala
   1 5
   AAG AAA CAA AAG TTT GCC AAC TCT TTG GAA GCC TT 1349
   Lys Lys Gln Lys Phe Ala Asn Ser Leu Glu Ala
   10 15
(2) INFORMATION POUR LA SEQ ID NO: 2:
   (i) CARACTERISTIQUES DE LA SEQUENCE:
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 2:
   CTGTTTCATT TGAAGCGCG 19
(2) INFORMATION POUR LA SEQ ID NO: 3:
   (i) CARACTERISTIQUES DE LA SEQUENCE:
      (A) LONGUEUR: 1226 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Kluyveromyces lactis
   (xi) DESCRIPTION DE LA SEQUENCE: SEQ ID NO: 3:
   ACGCGTGTCG ACGCTACCTA ATCCACAGGT GCCTTTGTAT AACTCCACTA ATTCGCTACA 60
   TTTTTCATCT CTTTGATTCG CTTTCATTAA TAAAATATTA CTATTCGAGT TGCTATAACT 120
   TTTAAGATCA TATACTTGTG TATATATGTT AGTTTGATGC TTTCTGCATT TTTCTTTATT 180
   TCCCTATAGT TTCTTCTGAT ATTCTAATCT GGCGACAGAT ACTGGGACAA ACACGTAAAT 240
   CACACACCAT GTCACGTGGT TAGAGAACAA AAGTGAAATA TGTGTTGGTG ACTGAATAAA 300
   ATGATGAAGG GTGTTTTGCT TAAAAACGGT GAATAAGTTA CCCGGCTGAT CTGAATAGAA 360
   TCGATTTTCT GCTTCTCAGC TTCCTATAAT CAAGAAAAAC GCCATGACCG GGTAACATGA 420
   ATTTTTGGGT GTCGTTATAC AGAGCATTGC CATCGACAAT GAACCAGGAT GCAATCTAAG 480
   GTGAATTTGA ATAAAATATA GCTCCATCTA CGACCAGGCA AGGATCACTG GAAAGTCATC 540
   CCGCATAAAC TACCCGGTTG TCCATCCGAT AAAATGAGTG CCATCTATAA ATCACTTTCT 600
   TTCTTACAAA CTAGTCTGTT TTTTCCTCCA TTATGTAACC ATTTAAACGT TAATTCCCCT 660
   AATGCGTCTG AATTACTCTC TGTTCAAAAC AAGAAACGAA AATCCGTACT CACAAACCAG 720
   CGATTCCCAT ACATGCATAC AATGATTTCA TAACAGTTTG ACTATTGCTC AGCAGGCGTG 780
   AATCAACACT TTGGCCCTGT CTAACGTTTA CGTATGCTAT GTTCTTCTCT CTTAGTTGTA 840
   TTGGGTTTTT CCGTGGCTTT CCTCATTTCC TACGGCCATT TCTCCCTTTC CCAGATTTTT 900
   TTCTTTCTTT GTTCTTCTTC CTGTTATATA ATCCGGATTT ATCACAGTAT ACAACTTTTC 960
   AAACTTTTGA TTTATTCAGA ATTGGAATGA TGAAATAATA TATTTGGTAT ATATATATAA 1020
   ATCAAAGGAA AAAGGTTAAC ATTGAGTTTT ACAAAGTGTT TTCTTCTTTT GCCAATTCAG 1080
   CTAAATTCTT AATCGGATCT AATTTTTGTT TAGTAAGATT CTTTATTTTG AGAAGGTTAT 1140
   TGTCTATTCT AATCTTGGTT ATTTCATTCA CCAGGAAACA GTAATTACAC TCAAGTAATT 1200
   TACCTTATAA ATCCTACTAC AAGCTT 1226